# EUROPEAN PATENT SPECIFICATION

(11) **EP 0 734 696 B1**
(45) Date of publication and mention of the grant of the patent: **29.08.2001**
(21) Application number: 96830151.5
(22) Date of filing: 26.03.1996
(51) Int. Cl.: A61C 19/00, A61L 2/24, A61L 2/18

(54) **A device for the collection and drainage of polluted liquids from dental instruments during or after the sterilization of the instruments and for holding the latter during sterilization**
Vorrichtung zum Sammeln und zur Entleerung von verschmutzten Flüssigkeiten aus zahnärztlichen Instrumenten während bzw. nach der Sterilisierung der Instrumente und zum Halten während der Sterilisierung
Dispositif de collecte et de drainage de liquides pollués dans les instruments dentaires pendant ou après la stérilisation des instruments et pour maintenir ceux-ci pendant la stérilisation

(30) Priority: 27.03.1995 IT BO950049 U
(43) Date of publication of application: 02.10.1996
(73) Proprietor: CASTELLINI S.p.A., I-40013 Castel Maggiore (Bologna) (IT)
(72) Inventor: Castellini, Franco, 40123 Bologna (IT)
(74) Representative: Lanzoni, Luciano

(56) References cited:
- EP-A- 0 321 415
- EP-A- 0 345 228

## Description

Dental unit with device for the collection and drainage of polluted liquids from dental instruments during or after the sterilization of the instruments and for holding the latter during sterilization

The present invention relates to a Dental unit with device for the collection and drainage of polluted liquids from dental instruments during or after the sterilization of the instruments and also for holding the latter during sterilization.

On account of the very high standards of hygiene required in dental surgeries, it is now normal practice to regularly sterilize dental instruments and handpieces.

One method of sterilizing dental equipment, used preferably when the -dental unit is not in operation, envisages an intermittent cycle (for example, see patent applications EP - 111.249 and EP -317.521, the latter being by the same Applicant as the present), during which the mains water supply to the handpieces is turned off and sterilizing liquid is fed into the handpiece pipes through a "dedicated" branch of the system equipped with an independent tank. After a defined time, which depends on the degree of sterilization required and on the amount of sterilizing liquid used, the water supply is turned on again and the polluted waste liquid is rinsed out through the handpieces themselves.

To recover the liquid and avoid dripping during the feeding in or rinsing out of the sterilizing liquid, the instruments, before the sterilization procedure is started, are placed in a specially designed container where the sterilizing liquid, polluted by the materials removed from the handpieces, collects.

Patent publication DE - 3.611.327 discloses an apparatus for holding handpieces and collecting the polluted sterilizing liquid, structured in such a way that it can be placed on a cuspidor integral with the central mounting assembly of the dental unit and in such a way that the sterilizing or rinsing liquid overflowing out of an annular chamber or trap made in the containing element itself drains off into the cuspidor.

The disadvantage of this solution, however, is that an important operation such as sterilization is carried out in the cuspidor where the risk of bacterial contamination is always high. Moreover, in order to empty it completely at the end of the sterilization cycle, the annular chamber must be removed from the unit and this considerably increases the time needed to end the sterilization cycle.

Another solution is described in patent publications IT - 212.161 and EP - 321.415 by the same Applicant as the present, which disclose an apparatus consisting of a container whose base has a projection that slots into the holder for a rinsing tumbler where there is a recess with a seat for the tumbler and a drainage hole for the supply pipe of the tumbler. The top of the apparatus has holes designed to receive the handpieces whose water pipes are to be sterilized, whilst the bottom has a hole for the direct drainage, i.e. without collection, of the polluted sterilizing liquid to the drain of the tumbler itself or, alternatively, a hole leading to an indirect drain that may be connected to an external suction unit built into the dental unit in order to allow it to be indirectly sterilized.

The disadvantage of these solutions is that the container where the liquid collects is always a clumsy accessory that remains separate from the rest of the dental unit and that must be removed when the dental unit is being used.

The aim of the present invention is to overcome the disadvantages described above. This aim is achieved in a device for the collection and drainage of polluted liquids from dental instruments during or after the sterilization of the instruments that is not only safe, reliable and independent of the units for the supply and collection of liquids normally used during treatment but also ergonomically structured in such a way as not to encumber the dental unit or hamper the movements of the dental surgeon.

The technical characteristics of the invention are laid out in the claims below and the advantages of the disclosure are apparent from the detailed description which follows, with reference to the accompanying drawings, which illustrate a preferred embodiment of the invention by way of example and in which:

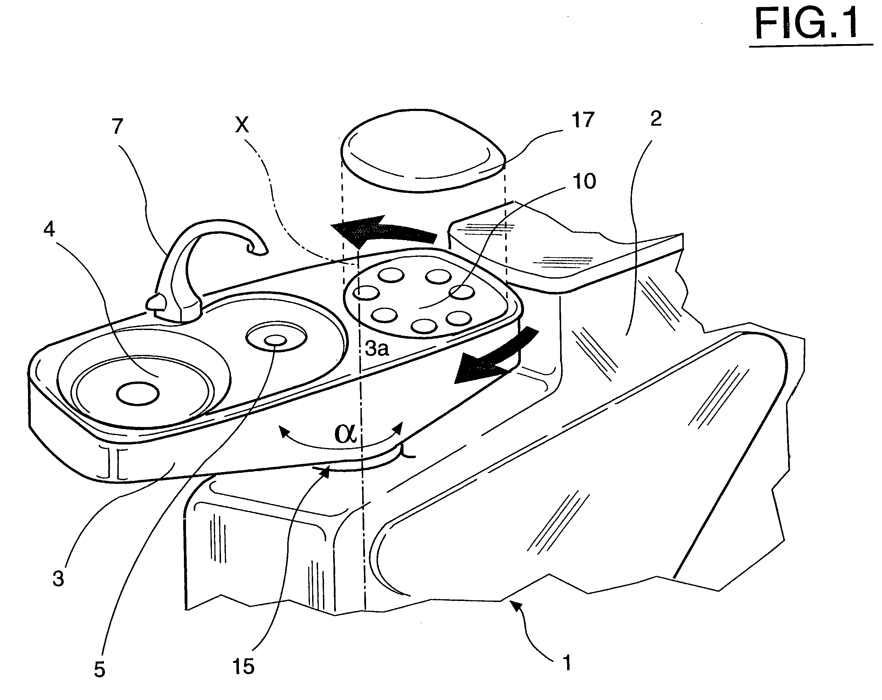

- Fig. 1 is a top perspective view of the polluted liquid collection and drainage device disclosed by the present invention, fitted to a dental unit, with some parts cut away in order to better illustrate others;

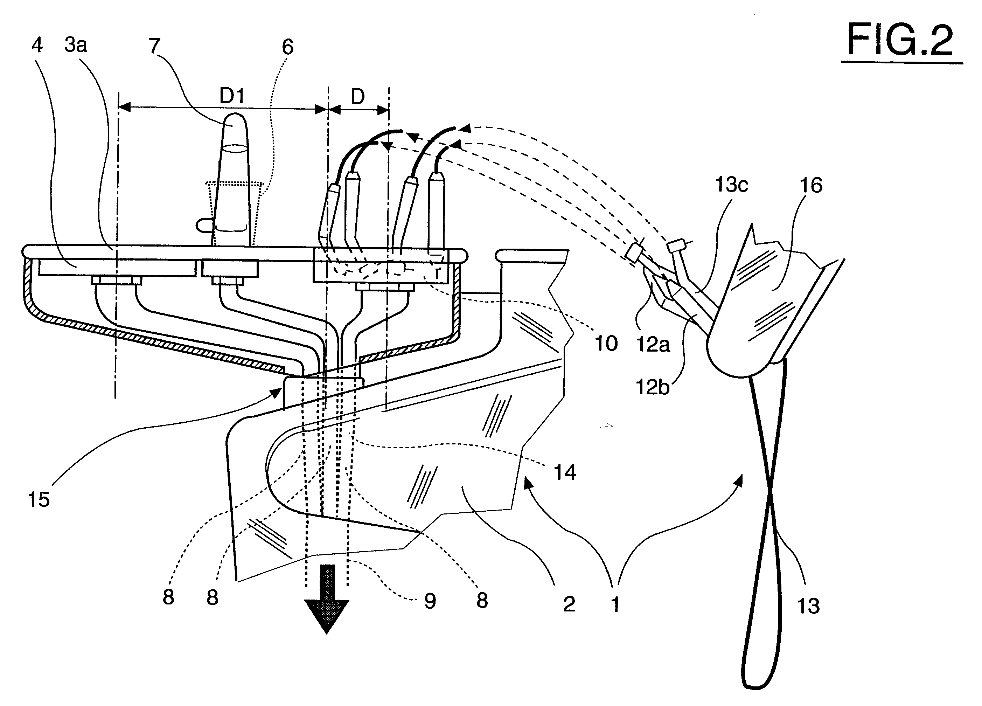

- Fig. 2 is side view of a part of the dental unit shown in Fig. 1, namely a base of an upper portion containing the device disclosed, with some parts in cross section in order to better illustrate others With reference to the accompanying drawings, in particular Fig. 1, the device disclosed allows the collection and drainage of polluted liquids from dental instruments 12 such as handpieces of known type (for example, a syringe 12a, a micromotor 12b and a turbine 12c). The device also holds the handpieces 12 while they are being sterilized.

The device is applied to dental units 1 including, amongst other things (see Figs. 1 and 2) a main base 2 with an upper portion 3 equipped with a cuspidor 4 and a plate 5 to support a tumbler 6 (shown by a dashed line) placed under a pipe which delivers a liquid (usually water).

The cuspidor 4 and the plate 5 have first drainage pipes 8 extending downwards into an area below the dental unit 1 and flowing together into a main drain 9.

Returning now to the device, this consists of a container 10 having a lid 11 with holes in it the holes being adapted to receive the instruments 12 are placed at rest so as to allow the drainage of the polluted liquids and of the rinsing liquid delivered by corresponding supply hoses 13 connected to each handpiece 12.

As is clearly visible in Figs. 1 and 2, the container 9 forms an integral part of the upper portion 3 of support and liquid collection and is connected independently to the main drain 9 through a second drainage pipe 14.

In short, the aim of the solution disclosed by the present invention is to create for the dental unit 1 an upper portion 3 with the purpose-designed container 10 for collecting and draining off polluted and rinsing liquids and built into the upper portion 3 together with the latter's other standard fittings (namely, cuspidor 4 and plate 5). In order to achieve this, the upper portion 3 is mounted by the main base 2 and can be partially swivelled about its vertical axis, labelled X in Fig. 2 (see also arrows F), on a swivel mounting 15. The mounting 15 is the only part that is in contact with the main base 2, the upper portion 3 being substantially "V" shaped and its top, horizontal surface 3a accommodating the container 10, the plate 5 and the cuspidor 4.

The container 10 and the cuspidor 4 are located at opposite ends of the horizontal surface 3a of the upper portion 3 and are separated by the plate 5 on which the rinsing tumbler 6 stands. The distance, labelled D, between the container 10 and the abovementioned vertical axis of rotation X of the upper portion 3 is less than the distance, labelled D1, between the said axis of rotation and the cuspidor 4.

This particularly ergonomic structure and layout of the different items allows the dental surgeon to turn the upper portion 3, according to need, either to move the cuspidor 4 closer to the patient or to move the container 10 closer to a tablet 16 carrying the handpieces 12 so that the latter can easily be moved from the tablet into the container 10, as shown by the dashed line in Fig. 2.

Obviously, it is the cuspidor 4 which must be moved the greater distance to be close to the patient so that he/she does not have to lean over too far to spit out the mouth wash. This explains the greater distance from the axis of rotation X and is also the reason why the upper portion 3 can rotate about its axis X by an angle A greater than 90°.

Inside the upper portion 3, the aforesaid first and second drainage pipes 8 and 14 flow together into the main drain 9 (shown by a dashed line in Fig. 2) at a point downstream of the swivel mounting 15 which joins the upper portion 3 to the main base 2. Obviously, the drainage pipes 8 and 14 must be structured and arranged in such a way that they do not interfere with the upper portion 3 while it is being turned.

The numeral 17 in Fig. 1 indicates a cover to be placed on the container 10 in order to close it when it is not being used to sterilize the dental instruments.

A device structured in the manner described above therefore achieves the set aims, that is to say, it constitutes a device which collects and drains off liquids while at the same time holding the instruments to be sterilized and which is built into that part of a dental unit where separate attachments used for the same purpose were previously applied.

Besides improving the general appearance of the dental unit and reducing the latter's overall dimensions, the device also guarantees safety in the drainage system, preventing sterilizing and polluted liquid from overflowing onto the areas where the patient and the dental surgeon normally come into contact with the dental unit.

The invention described can be subject to modifications and variations without thereby departing from the scope of the inventive concept. Moreover, all the details of the invention may be substituted by technically equivalent elements.

## Claims

1. Dental unit with device for collecting and draining off polluted liquids from dental instruments during or after the sterilisation of the instruments and also for holding the instruments themselves during sterilization, the said dental unit (1) comprising a main base (2) which mounts an upper portion (3) equipped with a cuspidor (4) and a plate (5) for supporting a tumbler (6) placed under a pipe which delivers a liquid; the said cuspidor (4) and plate (5) having first drainage pipes (8) flowing together into a main drain (9) located in an area below the dental unit (1); the said device consisting of at least one container (10) having a lid (11) with holes in it, the holes being adapted to the receive instruments (12) at rest so as to allow the drainage of the polluted liquids and of the rinsing liquid delivered by corresponding supply hoses (13) connected to each handpiece (12), and being **characterized in that** the container (10) forms an integral part of the upper portion (3) of support and liquid collection and is connected to the said main drain (9) through a separate second drainage pipe (14).

2. The device according to claim 1, **characterized in that** the said upper portion (3) stands on the main base (2) and can be partially swivelled about its vertical axis (X) on a swivel mounting (15).

3. The device according to claim 2, **characterized in that** the said upper portion (3) can rotate about its vertical axis (X) by an angle (A) greater than 90°.

4. The device according to claim 1, **characterized in that** the container (10) and the cuspidor (4) are located at opposite ends of the upper portion (3), separated from each other by the plate (5) on which the tumbler (6) stands, at a distance (D) from the said vertical axis of rotation (X) of the upper portion (3) that is less than the distance between the said axis of rotation and the cuspidor (4).

5. The device according to claim 1, **characterized in that** the said upper portion (3) stands on the main base (2) and can be partially swivelled about its vertical axis (X) on a mounting (15) and in that the said first drainage pipe (8) and second drainage pipe (14) flow together into the main drain (9) at a point downstream of the swivel mounting (15) which joins the upper portion (3) to the main base (2).

6. The device according to claim 5, **characterized in that** the said upper portion (3) can rotate about its vertical axis (X) by an angle (A) greater than 90°.

## Patentansprüche

1. Dentaleinheit mit einer Vorrichtung zum Sammeln und Entleeren von verschmutzten Flüssigkeiten aus zahnärztlichen Instrumenten während bzw. nach der Sterilisierung der Instrumente und zum Halten der Instrumente selbst während der Sterilisierung, wobei die genannte Dentaleinheit (1) einen Hauptsockel (2) enthält, welcher einen oberen Teil (3) trägt, letzterer ausgestattet mit einem Speibecken (4) und einer Abstellplatte (5) zum Halten eines Spülbechers (6), angeordnet unter einer Leitung zur Zufuhr von Flüssigkeit; wobei das genannte Speibecken (4) und die genannte Abstellplatte (5) erste Ablassleitungen (8) aufweisen, die zusammen in eine in dem Bereich unterhalb der Dentaleinheit (1) angeordnete Hauptablassleitung (9) münden; wobei die genannte Vorrichtung aus wenigstens einem Behälter (10) besteht, der einen Deckel (11) mit eingearbeiteten Öffnungen hat, wobei die Öffnungen dazu bestimmt sind, die Instrumente (12) aufzunehmen und in einer Ruhestellung zu halten, so dass das Ablassen der verschmutzten Flüssigkeiten und der von entsprechenden Schläuchen (13) zugeführten Spülflüssigkeiten erlaubt ist, die an jedes Handstück (12) angeschlossen sind, **dadurch gekennzeichnet,** dass der Behälter (10) einen ergänzenden Teil des oberen Teils (3) zum Tragen und Sammeln der Flüssigkeit bildet und über eine getrennte zweite Ablassleitung (14) an die genannte Hauptablassleitung (9) angeschlossen ist.

2. Vorrichtung nach Patentanspruch 1, **dadurch gekennzeichnet,** dass der genannte obere Teil (3) auf dem Hauptsockel (2) steht und auf einer Schwenkhalterung (15) teilweise um seine vertikale Achse (X) geschwenkt werden kann.

3. Vorrichtung nach Patentanspruch 2, **dadurch gekennzeichnet,** dass der genannte obere Teil (3) sich um seine vertikale Achse (X) drehen kann, und zwar um einen Winkel (A), der grösser ist als 90°.

4. Vorrichtung nach Patentanspruch 1, **dadurch gekennzeichnet,** dass der Behälter (10) und das Speibecken (4) an den entgegengesetzten Enden des oberen Teils (3) angeordnet sind, voneinander getrennt durch die Abstellplatte (5), auf welcher der Spülbecher (6) steht, und zwar um einen Abstand (D) von der genannten vertikalen Drehachse (X) des oberen Teils (3), welcher geringer ist als der Abstand zwischen der genannten Drehachse und dem Speibecken (4).

5. Vorrichtung nach Patentanspruch 1, **dadurch gekennzeichnet,** dass der genannte obere Teil (3) auf dem Hauptsockel (2) steht und auf einer Schwenkhalterung (15) teilweise um seine vertikale Achse (X) gedreht werden kann, und dadurch, dass die genannte erste Ablassleitung (8) und die genannte zweite Ablassleitung (14) zusammen in der genannten Hauptablassleitung (9) münden, und zwar an einem Punkt stromabwärts der Schwenkhalterung (15), welche den oberen Teil (3) mit dem Hauptsockel (2) verbindet.

6. Vorrichtung nach Patentanspruch 5, **dadurch gekennzeichnet,** dass sich der genannte obere Teil (3) sich um seine vertikale Achse (X) drehen kann, und zwar um einen Winkel (A), der grösser ist als 90°.

## Revendications

1. Unité dentaire avec dispositif de collecte et de drainage de liquides pollués dans les instruments dentaires pendant ou après la stérilisation des instruments et pour maintenir ceux-ci pendant la stérilisation, ladite unité dentaire (**1**) comprenant une base principale (**2**) sur laquelle est montée une partie supérieure (**3**) équipée d'une cuvette (**4**) et d'une plaque (**5**) pour le support d'un gobelet (**6**) posé sous un conduit qui délivre un liquide ; lesdites cuvette (**4**) et plaque (**5**) étant dotées de premiers conduits de drainage (**8**) qui convergent dans un conduit principal de drainage (**9**) situé dans une zone au-dessous de l'unité dentaire (**1**) ; ledit dispositif consistant en au moins un récipient (**10**) ayant un couvercle (**11**) présentant une série d'orifices prévus pour recevoir et maintenir des instruments (**12**) afin de permettre le drainage des liquides pollués et du liquide de rinçage distribué par des conduits d'alimentation (**13**) correspondants reliés à chacune des pièces à main (**12**), et **étant caractérisé en ce que** le récipient (**10**) fait partie intégrante de la partie supérieure (**3**) de support et de collecte du liquide et est relié audit conduit principal de drainage (**9**) par l'intermédiaire d'un deuxième conduit de drainage (**14**).

2. Le dispositif selon la revendication 1, **caractérisé en ce que** ladite partie supérieure (**3**) repose sur la base principale (**2**) et peut partiellement pivoter autour de son axe vertical (**X**) sur une articulation (**15**).

3. Le dispositif selon la revendication 2, **caractérisé en ce que** ladite partie supérieure (**3**) peut pivoter autour de son axe vertical (**X**) d'un angle (**A**) supérieur à 90°.

4. Le dispositif selon la revendication 1, **caractérisé en ce que** le récipient (**10**) et la cuvette (**4**) sont situés à des extrémités opposées de la partie supérieure (**3**) et sont séparés l'un de l'autre par la plaque (**5**) de support du gobelet (**6**), à une distance (**D**) dudit axe de rotation vertical (**X**) de la partie supérieure (**3**) inférieure à la distance entre ledit axe de rotation et la cuvette (**4**).

5. Le dispositif selon la revendication 1, **caractérisé en ce que** ladite partie supérieure (**3**) repose sur la base principale (**2**) et peut partiellement pivoter autour de son axe vertical (**X**) sur une articulation (**15**) et en ce que lesdits premiers conduits de drainage (**8**) et ledit deuxième conduit de drainage (**14**) convergent dans le conduit principal de drainage (**9**) en un point situé en aval de l'articulation (**15**) qui unit la partie supérieure (**3**) à la base principale (**2**).

6. Le dispositif selon la revendication 5, **caractérisé en ce que** ladite partie supérieure (**3**) peut pivoter autour de son axe vertical (**X**) d'un angle (**A**) supérieur à 90°.
